Europäisches Patentamt

European Patent Office

Office européen des brevets

(19)

(11) Numéro de publication : **0 412 865 B1**

## (12) FASCICULE DE BREVET EUROPEEN

(45) Date de publication du fascicule du brevet :
29.09.93 Bulletin 93/39

(51) Int. Cl.⁵ : **A61K 7/00**

(21) Numéro de dépôt : **90402018.7**

(22) Date de dépôt : **12.07.90**

(54) **Gel aqueux contenant en suspension des sphéroides d'une substance lipidique solide non hydrophile.**

(30) Priorité : **12.07.89 FR 8909421**

(43) Date de publication de la demande :
**13.02.91 Bulletin 91/07**

(45) Mention de la délivrance du brevet :
**29.09.93 Bulletin 93/39**

(84) Etats contractants désignés :
**AT BE CH DE DK ES FR GB GR IT LI NL SE**

(56) Documents cités :
EP-A- 0 006 724
EP-A- 0 376 852

(56) Documents cités :
DE-A- 2 725 924
FR-A- 2 218 086
GB-A- 543 309
GB-A- 2 204 792

(73) Titulaire : **L'OREAL**
**14, Rue Royale**
**F-75008 Paris (FR)**

(72) Inventeur : **Kauffmann, Myriam**
**25, Boulevard des Belges**
**F-69006 Lyon (FR)**

(74) Mandataire : **Stalla-Bourdillon, Bernard et al**
**CABINET NONY & CIE 29, rue Cambacérès**
**F-75008 Paris (FR)**

## Description

La présente invention a pour objet une composition cosmétique sous forme d'un gel aqueux contenant en suspension de petites sphères ou sphéroïdes d'une substance lipidique, solide non hydrophile, fondant par application sur le corps, ces sphéroïdes pouvant être chargés en au moins un composant cosmétique liposoluble ou non liposoluble.

De nombreuses compositions cosmétiques sont formulées sous forme de gel et l'on peut notamment mentionner les démaquillants, les toniques, les produits amincissants, les compositions anti-solaires et après-soleil, les gels capillaires, les produits d'hygiène buccale tels que les dentifrices ainsi que certains produits de maquillage tels que les mascaras.

Les formulations sous forme de gel sont tout particulièrement recherchées du fait de leur apport en eau conférant une sensation agréable de fraîcheur sur la peau et la muqueuse buccale et également du fait que les gels par rapport à d'autres formulations telles que les émulsions, ne confèrent pas un aspect gras à la peau ni aux cheveux.

Les gels présentent toutefois certains inconvénients dans la mesure où ils ont notamment un effet désséchant sur la peau, conduisant à un certain manque de confort. Par ailleurs, ils sont généralement mal supportés par les personnes présentant une peau à tendance sèche, voir même normale.

Très peu d'études ont été jusqu'ici entreprises en vue d'essayer de remédier à ces inconvénients de telle sorte que pour certaines applications on a souvent préféré remplacer les gels par des émulsions notamment du type huile-dans-l'eau. Toutefois celles-ci manquent de fraîcheur et confèrent à la peau un aspect gras et luisant. En outre, les agents émulsionnants utilisés peuvent éventuellement provoquer des phénomènes d'irritation sur certaines peaux particulièrement sensibles.

Par ailleurs, certains composants cosmétiques ne peuvent être introduits en quantité importante dans les gels ou les émulsions sans les déstabiliser. Il s'agit notamment des parfums et des huiles essentielles.

En outre deux composants incompatibles ou interactifs dans un gel, une huile dispersée dans un gel, ou une émulsion huile dans l'eau classique, ne peuvent coexister, que ceux-ci soient contenus dans la même phase, ou l'un dans la phase externe et l'autre dans la phase interne huileuse.

Dans ce dernier cas, les constituants du compartiment huileux sont mobiles, et entrent en contact avec ceux de la phase externe au niveau de l'interface.

C'est le cas par exemple de certains composants cosmétiques qui doivent, pour conserver leurs propriétés ou rester stables, ne pas entrer en contact avec l'eau. Même s'ils sont incorporés dans des gouttelettes d'huile au sein d'un gel ou d'une émulsion, ils restent accessibles à l'eau, peuvent y migrer, s'y dissoudre ou être hydrolysés.

Après différentes études, on vient maintenant de constater que les inconvénients des gels à savoir notamment l'effet désséchant et le manque de confort pouvaient être résolus en incorporant, sous forme de suspension, des sphéroïdes d'une substance lipidique, ceux-ci étant solides à la température ambiante mais fondant sous l'effet de l'élévation de température conséquente à l'application sur le corps, conférant ainsi aux gels de nouvelles propriétés notamment émollientes et lubrifiantes. Les sphéroïdes de substance lipidique permettent en effet d'empêcher l'effet de déssèchement de la peau et apportent une très agréable sensation de confort.

De plus, ces préparations biphasées du type huile dans l'eau, stables sans tensio-actif, dont le compartiment interne est solide, permettent de résoudre certains des problèmes mentionnés précédemment, tels que la stabilité des composants cosmétiques, de leur conservation, et de leur compatibilité entre eux.

La présente invention a donc pour objet une composition cosmétique sous forme d'un gel aqueux contenant, en suspension dans la phase continue, des sphéroïdes d'une substance lipidique, solide non-hydrophile, fondant par application sur le corps, lesdits sphéroïdes étant chargés ou non chargés en un composant cosmétique et présentant un diamètre moyen des particules compris entre 50 et 10.000 µm et un point de fusion inférieur à 50°C.

Par l'expression "corps" telle qu'utilisée selon l'invention on doit entendre non seulement la peau mais également le cuir chevelu, les cheveux, les ongles, les dents ou encore la muqueuse buccale.

Selon l'invention, la phase continue du gel peut être de l'eau ou un mélange d'eau et d'un solvant organique hydroxylé tel que par exemple l'alcool éthylique, la glycérine, les glycols tels que le propylèneglycol ou les éthers de glycol tels que le monoéthyléther de diéthylèneglycol. Dans cette dernière forme de réalisation, le mélange aqueux contient au moins 50% d'eau.

Par l'expression "sphéroïdes" telle qu'utilisée selon l'invention on doit entendre de petites particules solides essentiellement sphériques.

Le diamètre moyen des sphéroïdes s'il peut être compris dans de très larges limites, comme indiqué ci-dessus, est néanmoins de préférence compris entre 100 et 3.000µm.

2

Le pourcentage en poids des sphéroïdes dans le gel est bien entendu fonction de l'effet recherché, un pourcentage élevé conférant un effet adoucissant et lubrifiant plus prononcé au gel.

Dans la pratique, le pourcentage en poids des sphéroïdes dans le gel est généralement compris entre 0,1 et 50% en poids mais de préférence entre 1,5 et 10% en poids.

Il est particulièrement important selon l'invention que le point de fusion des sphéroïdes soit inférieur à 50°C et de préférence compris entre 30 et 45°C de telle sorte qu'ils puissent fondre par simple application sur le corps, éventuellement par un léger effet de frottement ou de massage.

Parmi les substances lipidiques solides non hydrophiles particulièrement préférées, formant les sphéroïdes, on peut notamment mentionner les glycérides hémisynthétiques, certaines substances grasses naturelles ainsi que certains composés gras synthétiques.

Parmi les glycérides hémisynthétiques on peut notamment citer les triglycérides d'acides gras saturés linéaires de 8 à 18 atomes de carbone, à indice d'hydroxyle < 30 et indice d'iode < 3, et parmi ceux-ci, ceux vendus sous les dénominations "LIPOCIRE A"® ou "SUPPOCIRE"®par la Société GATTEFOSSE, et en particulier les suivants : SUPPOCIRE®AIM"; "AM"; "BM"; "CM"; "DM"; "AI"; "A"; "B"; "C"; et "D" ou ceux vendus par la Société DYNAMIT NOBEL sous les dénominations WITEPSOL®ou SOFTISAN®, et en particulier les suivants : "WITEPSOL®$H_{32}$"; "$H_{35}$"; " $H_{37}$"; "$H_{39}$"; et "$H_{42}$".

Parmi les substances naturelles, on peut citer les beurres ou fractions solides de corps gras végétaux tels que par exemple : le beurre de karité, le beurre de cacao, l'huile de coprah et ses dérivés.

Parmi les composés synthétiques, on peut citer les cires de silicone telles que les produits vendus par la Société WACKER sous la dénomination "Cire silicone VP 1622" ou "Silicone copolymer F 755".

Les substances lipidiques telles qu'énumérées ci-dessus, peuvent être utilisées seules ou sous forme d'un mélange et dans ce dernier cas, il est préférable de parler de domaine ou de zone de fusion, plutôt que de point de fusion.

Il est également possible selon l'invention d'utiliser des substances lipidiques ayant un point de fusion supérieur à 50°C mais dans ce cas, il est nécessaire d'avoir recours à la présence d'un adjuvant lipidique de zone de fusion plus basse pour ajuster le point de fusion du mélange.

Parmi ces substances lipidiques à point de fusion supérieur à 50°C, mais utilisables sous forme d'un mélange avec d'autres lipides de point de fusion plus bas, on peut citer des triglycérides tels que le SOFTISAN 154®vendu par la Société DYNAMIT NOBEL, les cires animales, végétales, minérales ou synthétiques ou leurs dérivés, et en particulier la cire d'abeille, le blanc de baleine, les cires de candellila, de carnauba, leurs dérivés, notamment hydrogénés, les paraffines, les ozokérites, le produit commercialisé sous le nom "ELFACOS $C_{26}$"®par la Société AKZO CHEMIE.

Parmi les adjuvants destinés à abaisser le point de fusion des corps gras fondant au-dessus de 50°C, on peut notamment mentionner les alcools gras saturés linéaires en $C_{14}$ et $C_{16}$, ou ramifiés en $C_{12}$ à $C_{24}$, des esters gras, des acides gras insaturés, des mélanges complexes de lipides tels que des huiles végétales, des huiles de silicone...

Il est en outre possible pour ajuster la consistance ou la viscosité de ces sphéroïdes, d'introduire dans le mélange, des argiles modifiées ou leur dispersion huileuse, des silices, des savons métalliques ou tout autre structurant des corps gras.

Selon une forme de réalisation particulièrement préférée de l'invention, les sphéroïdes de substance lipidique contiennent, dissous ou dispersés dans leur matrice, des composants cosmétiques liquides ou solides.

Selon cette forme de réalisation, la substance lipidique des sphéroïdes sert de support et de véhicule à des composants tels que des parfums, des huiles essentielles ou leurs constituants, des pigments, des charges, des colorants, des vitamines, des enzymes et diverses autres substances actives au sens de la cosmétique qui peuvent être présents à raison de 0,01 à 70% et de préférence de 1 à 40% par rapport au poids des sphéroïdes.

Il va de soi que lorsque l'on incorpore ou charge de tels composants cosmétiques dans les sphéroïdes de substance lipidique, il convient de choisir une substance lipidique appropriée de telle sorte qu'après granulation, les sphéroïdes se présentent toujours sous forme solide ayant une température ou zone de fusion inférieure à 50°C.

L'incorporation de parfums et d'huiles essentielles dans les sphéroïdes est particulièrement appropriée car il est bien connu que ceux-ci sont des facteurs de destabilisation des gels.

L'incorporation de parfums dans les sphéroïdes permet par ailleurs de les protéger des phénomènes d'oxydation, ceux-ci y étant particulièrement sensibles.

Cette forme de réalisation des gels selon l'invention par incorporation de sphéroïdes chargés en parfum, en huiles essentielles ou leurs constituants, permet de rendre possible la formulation de produits, à phase externe aqueuse, particulièrement riches en ces composés ce qui jusqu'à présent ne pouvait être obtenu.

Parmi les composants cosmétiques liposolubles qui peuvent être incorporés dans les sphéroïdes, on peut mentionner les vitamines ou pro-vitamines plus ou moins liposolubles, telles que les vitamines A et E et leurs esters, les esters de la vitamine C, les caroténoïdes, des molécules antiseptiques, antiacnéiques, ou anti-pelliculaires, des filtres UV, des kératolytiques tels que l'acide salicylique ou ses dérivés ou ses sels, des molécules agissant sur la pigmentation, sur l'inflammation telles que les esters de zinc ou de cuivre et d'acides gras, des esters gras émollients, des huiles d'origine minérale, animale, végétale ou synthétique, des substituts ou composants du sébum tels que le squalène, le squalane, les céramides, le cholestérol, des extraits biologiques ou des colorants du cheveu etc...

On peut bien entendu incorporer dans les sphéroïdes de substance lipidique, sous forme de solides dispersés, des composants cosmétiques qui y sont insolubles tels que par exemple des vitamines hydrosolubles comme la vitamine C, des molécules biologiques telles que des enzymes, et toute autre substance utilisée en cosmétique et sensible à l'hydrolyse et/ou à l'oxydation en milieu aqueux ainsi qu'aux radiations UV.

Dans le cas de substances sensibles à l'hydrolyse, les excipients triglycéridiques à indice d'hydroxyle inférieur à 5 seront préférés.

Dans le cas de substances sensibles à l'oxydation, les excipients triglycéridiques à indice d'iode inférieur à 2 seront préférés.

Il est également possible d'incorporer dans ces sphères des charges adoucissantes ou lubrifiantes pour la peau telles que le talc, le produit vendu sous la dénomination "ORGASOL"® par la Société ATO, ou au contraire des particules destinées à produire un frottement ou une abrasion de la peau ou des dents, telles que des poudres de polyéthylène ou d'autres matières plastiques, des débris végétaux, cellulosiques ou ligniques, ou des particules minérales tels que les poudres de silice.

Selon une variante de ce mode de réalisation de l'invention il est possible de préparer des gels contenant en suspension des sphéroïdes chargés à l'aide de composants cosmétiques différents ce qui présente un intérêt particulier dans le cas de composants cosmétiques incompatibles entre eux tels que des parfums ou des huiles insaturées et des métaux ou des oxydes métalliques.

Les sphéroïdes des gels selon l'invention peuvent également contenir à un faible pourcentage d'autres composants habituels des phases lipidiques cosmétiques tels que des antioxydants et conservateurs.

Enfin ces sphéroïdes peuvent contenir eux-mêmes d'autres sphères, capsules ou systèmes vecteurs moléculaires dont la taille peut aller jusqu'à quelques centaines de microns ou tout autre système microcapsulaire ou micromatriciel. La formule globale est alors pluricompartimentée, chaque compartiment étant immobilisé.

Les sphéroïdes des gels selon l'invention peuvent être préparés par toutes méthodes conventionnelles de granulation ou de sphéronisation à chaud ou à froid.

Par exemple, par solidification par le froid de gouttelettes de la substance lipidique fondue, maintenue en mouvement dans un liquide non solvant tel que l'eau, ou par refroidissement par un gaz froid de gouttelettes de la substance lipidique pulvérisée, projetée sur un disque rotatif, ou extrudée, ou par granulation à chaud autour d'un noyau solide dans une turbine ou par lit d'air fluidisé, ou dans un mélangeur planétaire, par passage à travers une filière ou une grille vibrante, par moulage, coulage ou injection à chaud ou sous pression dans des moules, ou par découpage ou division d'une masse lipidique solide.

Les méthodes de préparation à froid, ou requérant peu d'échauffement, sont généralement préférées lorsque les composants cosmétiques inclus dans la substance lipidique sont fragiles vis-à-vis de la température ou de l'oxydation. Le refroidissement des gouttelettes fondues est obtenu de préférence par un gaz plutôt que par l'eau lorsque les substances cosmétiques qu'elles contiennent sont sensibles à la présence d'eau.

Selon le procédé choisi et les paramètres de fabrication, les sphéroïdes obtenus sont parfaitement calibrés ou de distribution de taille ou de diamètre plus ou moins étalé.

Les gels selon l'invention sont obtenus selon les méthodes conventionnelles à l'aide d'un agent gélifiant, généralement présent à une concentration comprise entre 0,02 et 70% en poids par rapport au poids total du gel (y compris les sphéroïdes chargés ou non chargés). Parmi les agents gélifiants particulièrement appropriés pour la formation des gels selon l'invention on peut notamment mentionner les polymères carboxyvinyliques tels que le CARBOPOL 940®, neutralisés par une base minérale ou organique (soude ou triéthanolamine), les gélifiants polysaccharidiques tels que les alginates, les gommes de xanthane, les dérivés de la cellulose, la gélatine et les gélifiants minéraux tels que les bentones ou les silices modifiées.

Les gels selon l'invention peuvent également contenir dans la phase aqueuse continue divers adjuvants cosmétiquement acceptables hydrosolubles et en particulier des colorants, des agents hydratants, des extraits biologiques, des vitamines ou des acides aminés, ou bien encore des adjuvants cosmétiquement acceptables non hydrosolubles tels que des charges ou des pigments.

Les gels selon l'invention peuvent également contenir dans la phase aqueuse continue des systèmes vecteurs tels que des liposomes ou nanocapsules ou tout autre système microcapsulaire ou non, ou matriciel ou micromatriciel.

On va maintenant donner à titre d'illustration plusieurs exemples de préparation des sphéroïdes chargés ou non chargés ainsi que des exemples de gels selon l'invention.

Procédé général de préparation des sphéroïdes.

L'ensemble substance(s) lipidique(s) + actif(s) est préparé à chaud. Les composants solides de la substance lipidique sont fondus à une température supérieure de 2 à 3°C à celle de la substance lipidique du plus haut point de fusion. Puis sont ajoutés les autres composants, en commençant par les moins fragiles.

Le mélange est maintenu à une température supérieure de 2 à 3°C à celle de sa zone de solidification. Les composants fragiles ou volatils sont ajoutés en dernier. Les solides sont ajoutés à la fin dans le mélange fondu, et maintenus sous agitation. Ils peuvent être éventuellement "empâtés" par une fraction de la substance lipidique fondue avant leur incorporation à la totalité du mélange.

Lorsque le mélange est homogène, la masse fondue est coulée lentement dans de l'eau portée à la même température. L'ensemble est maintenu sous agitation par un système rotatif pendant quelques minutes. La masse lipidique fondue se disperse alors en gouttelettes.

La vitesse de rotation du système conditionne la taille des sphéroïdes obtenus : le diamètre des particules diminue quand la vitesse de rotation augmente.

La dispersion obtenue est rapidement refroidie par addition d'eau à une température d'environ 0°C ou par immersion dans un bain d'eau glacée à une température inférieure à 0°C. L'agitation est arrêtée lorsque les gouttelettes sont solidifiées. Les sphéroïdes, éventuellement chargés, sont ensuite séparés par filtration puis séchés à température ambiante.

Selon ce mode opératoire on a préparé des sphéroïdes, non chargés et chargés en diverses substances, selon les exemples suivants :

1. Exemples de sphéroïdes non chargés.

A. Mélange de triglycérides hémisynthétiques en $C_8$-$C_{18}$
(vendu par la Société GATTEFOSSE sous la dénomination de "LIPOCIRE A")®........ 100g

Zone de fusion : 36-38°C

Taille moyenne des particules : 1 mm

B. - Triglycérides semi-synthétiques vendu par la Société GATTEFOSSE sous la dénomination "SUPPOCIRE CM"®   90g
- Huile de vaseline ...............   10g

Zone de fusion : 36-38°C

Taille moyenne des particules : 1,5 mm

2. <u>Exemples de sphéroïdes chargés en parfum.</u>

```
        A. Concentré de parfum................       30g
            "SUPPOCIRE AI"®de la Société GATTEFOSSE  29,748g
            "SUPPOCIRE DM"®de la Société GATTEFOSSE  40g
            D et C Red 17.......................     0,001g
            D et C Violet 2.....................     0,001g
            Conservateur........................     0,2g
            Butylhydroxytoluène.................     0,05g
```

<u>Zone de fusion</u> : 32-39°C.

<u>Taille moyenne des particules</u> : 1 mm

```
        B. Concentré de parfum.................      20g
            "SUPPOCIRE D"®de la Société GATTEFOSSE  63,75g
            Alcool myristique...................     10g
            Miglyol®gel de la Société DYNAMIT NOBEL  4g
            Nacre...............................      2g
            Butylhydroxytoluène.................     0,05g
            Conservateur........................     0,2g
```

<u>Zone de fusion</u> : 33-42°C.

<u>Taille moyenne des particules</u> : 2 mm

```
        C. Concentré de parfum.................      30g
            Alcool myristique...................     10g
            "SUPPOCIRE B"®de la Société GATTEFOSSE   50g
            "SOFTISAN 154"®
                de la Société DYNAMIT NOBEL ......... 9,948g
            D et C Red 17.......................     0,002g
            Butylhydroxytoluène.................     0,05g
```

<u>Zone de fusion</u> : 35-42°C.

<u>Taille moyenne des particules</u>: 500µm

3. <u>Exemple de sphéroïdes chargés en poudre abrasive.</u>

```
            "SUPPOCIRE DM"®de la Société GATTEFOSSE  85g
            Concentré de parfum.................      5g
            Poudre de silice....................     10g
```

<u>Zone de fusion</u> : 39-42°C

<u>Taille moyenne des particules</u> : 2 mm

#### 4. Exemple de sphéroïdes chargés en pro-vitamines.

| | |
|---|---|
| "SUPPOCIRE DM"®de la Société GATTEFOSSE | 55g |
| Alcool myristique...................... | 10g |
| Miglyol®gel de la Société DYNAMIT NOBEL | 9g |
| Acétate d' α-tocophérol............... | 5g |
| Nicotinate d' α-tocophérol........... | 5g |
| Extrait huileux de carotte........... | 15g |
| Parfum............................... | 1g |

<u>Zone de fusion</u> : 34-41°C

<u>Taille moyenne des particules</u> : 1 mm

#### 5. Exemples de sphéroïdes chargés en pigments.

| | |
|---|---|
| A. "SUPPOCIRE B"®de la Société GATTEFOSSE | 83,20g |
| Oxyde de fer brun.................... | 1,55g |
| Oxyde de fer ocre.................... | 1,55g |
| Dioxyde de titane.................... | 13,50g |
| Conservateur......................... | 0,20g |

<u>Zone de fusion</u> : 39-40°C

<u>Taille moyenne des particules</u> : 500µm

| | |
|---|---|
| B. "SUPPOCIRE AI"®de la Société GATTEFOSSE | 73,8g |
| Dioxyde de titane.................... | 20,0g |
| Oxyde de chrome...................... | 1,0g |
| Talc................................. | 5,0g |
| Conservateur......................... | 0,2g |

<u>Zone de fusion</u> : 37-39°C

<u>Taille moyenne des particules</u> : 200µm

#### 6. Exemple de sphéroïdes chargés en anti-pelliculaire.

| | |
|---|---|
| Cire de Silicone SLM 50553/1 de la Société WACKER................... | 99g |
| Zinc pyrithione...................... | 1g |

Zone de fusion: 35-40°C

Taille moyenne des particules: 0,2mm

7. Exemple de sphéroïdes chargés en menthol.

"SUPPOCIRE A'"® de la Société GATTEFOSSE        79,5g

Menthol codex ...........................        20g

Oxyde de chrome.........................         0,5g

Zone de fusion: 25-40°C

Taille moyenne des particules: 2,5mm

EXEMPLES DE GELS

8. Gels parfumés

A. "CARBOPOL 940"® de la Société GOODRICH        0,3g

Hydroxyde de sodium q.s.......pH 6,5

Collagène soluble....................           0,05g

Glycérine............................           4g

Filtre UV............................           0,2g

FD et C Blue 1.......................           0,004g

Conservateur.........................           0,02g

Sphéroïdes chargés en parfum

selon l'exemple 2A...................           8g

Eau q.s.p............................           100g

Le gel obtenu, limpide et bleuté, contient en suspension les sphéroïdes de couleur grise fortement parfumés. Par application sur la peau les sphéroïdes fondent très rapidement libérant le parfum. Le gel aqueux externe apporte confort et fraicheur et la substance lipidique des sphéroïdes a un effet adoucissant et émollient. De plus il se forme un film adhérent parfumé rémanent.

B. Gomme xanthane.......................        1,5g

Sorbitol solution à 70%..............           5g

Conservateur.........................           0,02g

Sphéroïdes chargés en parfum

selon l'exemple 2B ..................           6g

Eau q.s.p............................           100g

C. Silicate de magnésium................ 4,5g

   Glycérine........................... 6g

   Propylèneglycol..................... 2g

   Conservateur........................ 0,01g

   Sphéroïdes chargés en parfum

   selon l'exemple 2C.................. 7g

   Eau q.s.p........................... 100g

9. Gel nettoyant.

   "CARBOPOL 940" de la Société GOODRICH    0,3g

   Hydroxyde de sodium q.s..... pH = 7

   Glycérine........................... 8g

   Tensio-actif ("MIRANOL C2M"®

   de la Société MIRANOL).............. 0,3g

   Conservateur........................ 0,02g

   Sphéroïdes chargés en poudre abrasive

   selon l'exemple 3 .................. 8g

   Eau q.s.p........................... 100g

10. Gel de soin.

   "CARBOPOL 1342"®de la Société GOODRICH    0,3g

   Triéthanolamine q.s ........pH = 6,5

   Glycérine .......................... 5g

   Conservateur ....................... 0,01g

   Sphéroïdes chargés en pro-vitamines

   selon l'exemple 4.................. 5g

   Eau q.s.p.......................... 100g

11. Gel adoucissant.

   "CARBOPOL 940"®de la Société GOODRICH.    0,25g

   Triéthanolamine q.s...... pH = 6,5

   Glycérine........................... 4g

   Panthénol........................... 3g

   Sphéroïdes non chargés

   selon l'exemple 1B................. 6g

   Eau q.s.p.......................... 100g

12. Gel mascara.

| | |
|---|---|
| "CARBOPOL 910"® de la Société GOODRICH | 0,8g |
| Triéthanolamine q.s..........pH = 6,5 | |
| Glycérine............................ | 6g |
| Panthénol........................... | 4g |
| Sphéroïdes chargés en pigments selon l'exemple 5A................... | 3g |
| Sphéroïdes chargés en pigments selon l'exemple 5B................... | 1g |
| Eau q.s.p ......................... | 100g |

Ce mascara est introduit dans un réservoir, de préférence transparent, et équipé d'un dispositif de cisaillement. Ainsi lors de l'utilisation, la brosse, forcée à travers un essoreur de diamètre inférieur au sien, disperse par cisaillement les pigments dans le gel aqueux. Ceci permet de former une gaine régulière autour des cils.

13. Gel antipelliculaire pour le cuir chevelu.

| | |
|---|---|
| "CARBOPOL 940"® de la Société GOODRICH | 0,2g |
| Soude q.s.p. ................pH = 6,5 | |
| Conservateur q.s. | |
| Sphèroïdes chargés en zinc pyrithione selon l'exemple 6................... | 10g |
| Eau q.s.p. | 100g |

14. Gel gingival.

| | |
|---|---|
| "LAPONITE XLG"® de la Société LAPORTE INDUSTRIES Ltd.............. | 0,23g |
| Carboxyméthylcellulose de sodium.... | 0,38g |
| Glycérine......................... | 25g |
| Conservateur q.s. | |
| Saccharinate de sodium............. | 0,1g |
| Extrait propylène glycolique de rhubarbe | 10g |
| Sphéroïdes chargés en menthol selon l'exemple 7................... | 5g |
| Eau q.s.p. | 100g |

**Revendications**

1. Gel aqueux à usage cosmétique, caractérisé par le fait qu'il contient, en suspension dans la phase continue, des sphéroïdes d'une substance lipidique solide non hydrophile fondant par application sur le corps, lesdits sphéroïdes étant chargés ou non chargés en un composant cosmétique et présentant un diamètre moyen des particules compris entre 50 et 10.000 μm et un point de fusion inférieur à 50°C; les sphéroïdes chargés en une substance thérapeutique sont exclus.

2. Gel selon la revendication 1, caractérisé par le fait que la phase continue est de l'eau ou un mélange d'eau et d'un solvant organique hydroxylé, ledit mélange contenant au moins 50% d'eau.

3. Gel selon la revendication 1, caractérisé par le fait que le diamètre moyen des particules est de préférence compris entre 100 et 3.000µm.

4. Gel selon l'une quelconque des revendications 1 à 3, caractérisé par le fait que les sphéroïdes sont présents dans le gel en une proportion comprise entre 0,1 et 50% en poids et de préférence entre 1,5 et 10% par rapport au poids total du gel.

5. Gel selon l'une quelconque des revendications 1 à 4, caractérisé par le fait que les sphéroïdes ont un point de fusion compris entre 30 et 45°C.

6. Gel selon l'une quelconque des revendications 1 à 5, caractérisé par le fait que la substance lipidique solide non hydrophile des sphéroïdes est prise dans le groupe constitué par les triglycérides d'acides gras, saturés linéaires ayant de 8 à 18 atomes de carbone, les beurres ou fractions solides de corps gras végétaux et les cires de silicone.

7. Gel selon l'une quelconque des revendications 1 à 6, caractérisé par le fait que les sphéroïdes sont chargés en au moins un composant cosmétique, liquide ou solide et liposoluble ou non liposoluble.

8. Gel selon la revendication 7, caractérisé par le fait que les sphéroïdes sont chargés en un parfum, une huile essentielle, un pigment, une charge, une substance abrasive, un colorant et/ou une substance active cosmétique.

9. Gel selon l'une quelconque des revendications 7 et 8, caractérisé par le fait que les sphéroïdes sont chargés en au moins un ingrédient cosmétique à raison de 0,01 à 70% et de préférence de 1 à 40% en poids par rapport au poids des sphéroïdes.

10. Gel selon l'une quelconque des revendications précédentes, caractérisé par le fait que l'agent gélifiant est présent en une proportion comprise entre 0,02 et 70% en poids par rapport au poids total du gel.

11. Gel selon l'une quelconque des revendications précédentes, caractérisé par le fait que la phase continue du gel contient au moins un adjuvant cosmétique hydrosoluble tel qu'un colorant, un agent hydratant, un extrait biologique, une vitamine ou un acide aminé.

12. Gel selon l'une quelconque des revendications précédentes caractérisé par le fait que la phase continue du gel contient au moins un adjuvant cosmétique non hydrosoluble tel qu'une charge ou un pigment.

## Claims

1. Aqueous gel for cosmetic use, characterized by the fact that it contains, in suspension in the continous phase, spheroids of a water-insoluble solid lipid substance which melts on application to the body, the said spheroids being charged or not being charged with a cosmetic component and having a mean particle diameter of between 50 and 10,000 µm and a melting point of less than 50°C; spheroids charged with a therapeutic substance are excluded.

2. Gel according to Claim 1, characterised by the fact that the continuous phase is water or a mixture of water and a hydroxylated organic solvent, the said mixture containing at least 50 % of water.

3. Gel according to Claim 1, characterised by the fact that the mean particle diameter is preferably between 100 and 3000 µm.

4. Gel according to any one of Claims 1 to 3, characterised by the fact that the spheroids are present in the gel in a proportion of between 0.1 and 50 % by weight and preferably between 1.5 and 10 % relative to the total weight of the gel.

5. Gel according to any one of Claims 1 to 4, characterized by the fact that the spheroids have a melting point of between 30 and 45°C.

6. Gel according to any one of Claims 1 to 5, characterised by the fact that the water-insoluble solid lipid substance of the spheroids in chosen from the group consisting of linear saturated fatty acid triglycerides having from 8 to 18 carbon atoms, butters or solid fractions of vegetable fats and silicone waxes.

7. Gel according to any one of Claims 1 to 6, characterised by the fact that the spheroids are charged with at least one fat-soluble or fat-insoluble solid or liquid cosmetic component.

8. Gel according to Claim 7, characterised by the fact that the spheroids are charged with a perfume, an essential oil, a pigment, a filler, an abrasive substance, a colorant and/or an active cosmetic substance.

9. Gel according to any one of Claims 7 and 8, characterised by the fact that the spheroids are charged with at least one cosmetic ingredient in an amount of 0.01 to 70 % and preferably of 1 to 40 % by weight relative to the weight of the spheroids.

10. Gel according to any one of the preceding claims, characterised by the fact that the gelling agent is present in a proportion of between 0.02 and 70 % by weight relative to the total weight of the gel.

11. Gel according to any one of the preceding claims, characterised by the fact that the continuous phase of the gel contains at least one water-soluble cosmetic adjuvant such as a colorant, a moisturising agent, a biological extract, a vitamin or an amino acid.

12. Gel according to any one of the preceding claims, characterised by the fact that the continuous phase of the gel contains at least one water-insoluble cosmetic adjuvant such as a filler or a pigment.

## Patentansprüche

1. Wäßriges Gel zur kosmetischen Verwendung, dadurch gekennzeichnet, daß es als Suspension in der kontinuierlichen Phase Kügelchen aus einer festen, nicht-hydrophilen Lipidsubstanz enthält, die beim Aufbringen auf den Körper schmelzen, wobei diese Kügelchen mit einem kosmetischen Bestandteil beladen oder nicht beladen sind, und der mittlere Durchmesser der Teilchen zwischen 50 und 10 000 μm liegt und einen Schmelzpunkt von unter 50°C besitzen, wobei mit einer therapeutischen Substanz gefüllte Kügelchen ausgeschlossen sind.

2. Gel nach Anspruch 1, dadurch gekennzeichnet, daß die kontinuierliche Phase Wasser oder eine Mischung von Wasser und einem hydroxylierten organischen Lösungsmittel ist, wobei die Mischung mindestens 50% Wasser enthält.

3. Gel nach Anspruch 1, dadurch gekennzeichnet, daß der mittlere Durchmesser der Teilchen vorzugsweise zwischen 100 und 3 000 μm beträgt.

4. Gel nach einem der Ansprüche 1 bis 3, dadurch gekennzeichnet, daß die Kügelchen in dem Gel in einem Anteil zwischen 0,1 und 50 Gew.-% und vorzugsweise zwischen 1,5 und 10 Gew.-%, bezogen auf das Gesamtgewicht des Gels, vorhanden sind.

5. Gel nach einem der Ansprüche 1 bis 4, dadurch gekennzeichnet, daß die Kügelchen einen Schmelzpunkt zwischen 30 und 45°C besitzen.

6. Gel nach einem der Ansprüche 1 bis 5, dadurch gekennzeichnet, daß die feste, nicht-hydrophile Lipidsubstanz der Kügelchen aus der Gruppe bestehend aus linearen, gesättigten und 8 bis 18 Kohlenstoffatome enthaltenden Fettsäuretriglyceriden, Butterstoffen oder festen Fraktionen von pflanzlichen Fetten und Silikonwachsen ausgewählt ist.

7. Gel nach einem der Ansprüche 1 bis 6, dadurch gekennzeichnet, daß die Kügelchen mit mindestens einem flüssigen oder festen, lipidlöslichen oder nicht-lipidlöslichen kosmetischen Bestandteil beladen sind.

8. Gel nach Anspruch 7, dadurch gekennzeichnet, daß die Kügelchen mit einem Parfum, einem ätherischen Öl, einem Farbstoff, einem Füllstoff, einem Schleifstoff, einem Färbemittel und/oder einer kosmetisch aktiven Substanz beladen sind.

9. Gel nach einem der Ansprüche 7 und 8, dadurch gekennzeichnet, daß die Kügelchen mit mindestens einem kosmetischen Bestandteil in einer Menge von 0,01 bis 70 Gew.-% und vorzugsweise von 1 bis 40 Gew.-%, bezogen auf das Gewicht der Kügelchen, beladen sind.

10. Gel nach einem der vorhergehenden Ansprüche, dadurch gekennzeichnet, daß das Geliermittel in einer Menge zwischen 0,02 und 70 Gew.-%, bezogen auf das Gesamtgewicht des Gels, vorhanden ist.

11. Gel nach einem der vorhergehenden Ansprüche, dadurch gekennzeichnet, daß die kontinuierliche Phase des Gels mindestens einen wasserlöslichen kosmetischen Zusatzstoff wie einen Farbstoff, ein Feuchthaltemittel, einen biologischen Extrakt, ein Vitamin oder eine Aminosäure enthält.

12. Gel nach einem der vorhergehenden Ansprüche, dadurch gekennzeichnet, daß die kontinuierliche Phase des Gels mindestens einen nicht-wasserlöslichen kosmetischen Zusatzstoff wie einen Füllstoff oder einen Farbstoff enthält.